# EUROPEAN PATENT SPECIFICATION

(11) **EP 2 711 309 B1**
(45) Date of publication and mention of the grant of the patent: **24.08.2016**
(21) Application number: 12185271.9
(22) Date of filing: 20.09.2012
(51) Int. Cl.: B65D 83/38

(54) **Canister for a metered dose inhaler and method of producing such canister**
Behälter für einen Messdosierinhalator und Verfahren zur Herstellung des Behälters
Absorbeur pour un inhalateur de dose mesurée et procédé de production d'un tel absorbeur

(43) Date of publication of application: 26.03.2014
(73) Proprietor: Presspart Gmbh & Co. Kg, 34431 Marsberg (DE)
(72) Inventor: Neugebauer, Hans-Jürgen, 34414 Warnburg (DE)
(74) Representative: Uexküll & Stolberg

(56) References cited:
- EP-A1- 1 428 762
- WO-A1-2009/073012
- US-A- 5 211 317

## Description

The present invention relates to a canister for a metered dose inhaler for storing a propellant and a medicament under pressure and dispensing said medicament, and to a method of producing such a canister.

One frequent manner of administering therapeutic, prophylactic or diagnostic compounds, i.e. drugs and medicaments, to a patient in connection with the treatment of respiratory and nasal diseases, such as, e.g., asthma or chronic obstructive pulmonary disease (COPD), involves delivering these compounds to the patient in aerosol formulations. For this purpose, a low boiling point liquid or liquefied gas may advantageously be formulated with the respective compound or compounds and disposed under pressure in a closed container or canister. The canister also comprises a metering valve by means of which a predetermined quantity of the compound formulation can be metered and the corresponding dose can be dispensed as an inhalable cloud. In this regard, the low boiling point liquid or liquefied gas constitutes a propellant which is capable of expelling the formulation through the metering valve.

In actual use the canister is arranged in a device which provides a channel for guiding the formulation expelled from the metering valve into the mouth or nose of the patient, and which is often also designed for facilitating actuation of the metering valve. For example, the metering valve may include a valve stem extending from the metering valve and providing a conduit for passing the metered dose out of the canister, and the device may include a receptacle for receiving the canister and a nozzle block which receives the valve stem of a canister arranged in the receptacle. A portion of the canister opposite the metering valve and valve stem projects from the receptacle, so that the patient can depress this portion and the canister into the receptacle, thereby actuating the metering valve.

The combination of such a device with a canister including the propellant and compound formulation (typical pMDI formulation including the active pharmaceutical ingredient (API), where appropriate a cosolvent (e.g. alcohol), where appropriate surfactant/excipients and the propellant) is typically referred to as metered dose inhaler (MDI). In this context, the canister for a metered dose inhaler, i.e. for use in and as part of a metered dose inhaler, constitutes a cartridge-like component.

In order to maintain the propellant in the liquid state, a considerable pressure must be present in the interior of the canister. Consequently, the canister must be capable of safely withstanding such elevated pressures. Specifically, according to ICAO regulations the burst pressure of the canister must be at least 220 psi, i.e. 15.2 bar. As a safety measure, the bottom wall of the canister is typically formed into an inwardly domed shape, and the construction of the canister is chosen such that upon exceeding a particular pressure value somewhat below the admissible 15.2 bar burst pressure the inwardly domed shape is inverted to an outwardly domed shape, thereby increasing the interior volume of the canister and decreasing the interior pressure. This inversion of the inwardly domed bottom also indicates to the user that the interior pressure of the canister is at a dangerous level.

Known canisters for metered dose inhalers have a wall thickness which is constant or largely constant at least throughout their sidewall and their bottom wall. In this regard it is to be noted that the sidewall and the bottom wall are often integrally formed in one piece.

For example, for more than 50 years such canisters have been produced from aluminum or Al-Mg alloys such as, in particular, A1 5052 alloy, i.e. AlMg_{2.5}, or from stainless steel by means of deep drawing. Today, more than 90% of all canisters for metered dose inhalers are made from aluminum or Al-Mg alloys. Their wall thickness is in the range of 0.47 mm.

After use the canister is discarded and not recycled. In view of this as well as in view of the fact that aluminum is a very expensive material requiring a large amount of energy for its production and generating a lot of carbon dioxide in the production process, the above-mentioned constructional parameters were chosen in an attempt to reduce the amount of material used while at the same time providing the required pressure and burst characteristics. Nevertheless, it is still desirable to find a way to further decrease the costs of the canisters and the amount of energy consumed in their production.

Therefore, it is an object of the present invention to provide a canister for a metered dose inhaler which can be produced in a less expensive and less energy intensive manner, and a corresponding method.

This object is achieved by means of a canister for a metered dose inhaler having the features of claim 1 and by a method of producing a canister for a metered dose inhaler having the features of claim 14. Preferred embodiments of the canister and the method are the subject-matter of the respective dependent claims.

According to the invention, a canister for a metered dose inhaler for storing a propellant and a medicament under pressure and dispensing said medicament, i.e. a canister for use in a metered dose inhaler, comprises an interior cavity or volume which is defined by a tubular sidewall, a bottom wall and a top cover. As explained above, the interior cavity is adapted for receiving or contains a medicament in solution or suspension with a low boiling point propellant in a pressurized condition.

The tubular sidewall is preferably cylindrical and may advantageously have a circular cross section. Further, the entire canister is preferably symmetrical about the longitudinal axis. In any case, the tubular sidewall defines a longitudinal axis of the canister and its interior cavity. Thus, it is preferred that the sidewall extends in a straight manner along a longitudinal direction.

The bottom wall and the top cover close opposite longitudinal ends of the sidewall such that the sidewall extends along the longitudinal axis between the bottom wall and the top cover. Thus, in an orientation of the container in which the bottom wall points downwardly, the sidewall extends upwardly from the bottom wall, preferably perpendicularly or essentially perpendicularly. The sidewall may, for example, define a necked opening to which the cover portion is secured, e.g. by means of crimping. Details of the construction of the cover portion are not essential to the present invention. Thus, different cover portion arrangements are possible.

The sidewall and the bottom wall are preferably provided in the form of sheet metal, preferably an aluminum magnesium alloy, preferably aluminum alloy 5052, i.e. AlMg_{2.5}. Other possible materials include Al 3003 and Al 3004. Further, while the sidewall and the bottom wall are preferably bare, the sidewall and/or the bottom wall may also be coated on the inside and/or on the outside.

The bottom wall comprises a central inwardly domed or curved portion. In other words, in a top plan view of the bottom wall, the central domed or curved portion constitutes a concave portion. Preferably, the point of deepest depression of the central domed portion is located at the center of the bottom wall. It is also preferred for the central domed portion to be rotationally symmetric. In particular, the central domed portion may have a circular circumference and be symmetric about the center of the corresponding circle.

In connection with the central domed portion the word "central" is intended to indicate that the domed portion is not located at only one border of the bottom wall. Rather, the central domed portion either extend over the entire or substantially the entire bottom wall, or the central domed portion is completely surrounded by another portion of the bottom wall, as will be described later-on.

The sidewall has a wall thickness which is smaller than the wall thickness of the entire bottom wall or at least the central domed portion thereof. Specifically, the thickness of the bottom wall is in the range of 0.25 to 0.47 mm, and the thickness of the sidewall is in the range of 0.15 to 0.42 mm. In preferred embodiments the thickness of the bottom wall is in a range having a lower limit of 0.26 mm, preferably 0.28 mm, more preferably 0.3 mm, even more preferably 0.32 mm, still more preferably 0.34 mm, and most preferably 0.36 mm, and having an upper limit of 0.46 mm, more preferably 0.45 mm, even more preferably 0.44 mm, still more preferably 0.43 mm, and most preferably 0.42 mm. For example, in a preferred embodiment the thickness of the bottom wall is in the range of 0.36 to 0.42 mm. Further, in preferred embodiments the thickness of the sidewall is in a range having a lower limit of 0.15 mm, preferably 0.16 mm, more preferably 0.17 mm, even more preferably 0.18 mm, still more preferably 0.19 mm, and most preferably 0.2 mm, and having an upper limit of 0.41 mm, more preferably 0.4 mm, even more preferably 0.39 mm, still more preferably 0.38 mm, still more preferably 0.37 mm, and most preferably 0.36 mm. For example, in a preferred embodiment the thickness of the sidewall is in the range of 0.15 to 0.36 mm.

It has been found surprisingly that the required burst pressure characteristics can still be achieved with lower thickness values than used in the prior art canisters. In particular, it is possible to considerably reduce the thickness of the sidewall as compared to the bottom wall if values from the specified ranges are used. Due to the reduced material thickness considerable savings in the amount of material used for the production of the canisters are achieved, thereby reducing the costs and the amount of energy consumed. At the same time, the carbon dioxide footprint of the canisters is advantageously decreased considerably, in particular for canisters comprising aluminum.

In a preferred embodiment the sidewall and the bottom wall are integrally formed in one piece. Such a canister may be produced by deep drawing a cup-shaped component including the sidewall and the bottom wall.

In a preferred embodiment the thickness of the sidewall is in the range of 0.18 to 0.36 mm, preferably in the range of 0.2 to 0.36 mm, even more preferably in the range of 0.22 to 0.36 mm, still more preferably in the range of 0.24 to 0.36 mm, still more preferably in the range of 0.26 to 0.36 mm, even still more preferably in the range of 0.28 to 0.36 mm, and most preferably in the range of 0.3 to 0.36 mm. In further preferred embodiments the upper limit of these preferred ranges may also be 0.34 mm, 0.32 mm or 0.3 mm.

In a preferred embodiment the bottom wall comprises an annularly closed portion surrounding the central domed portion and disposed between the central domed portion and the sidewall, i.e. the longitudinal end of the sidewall to which the bottom wall is connected. Preferably, this annularly closed portion is rotationally symmetric and may exhibit, in particular, circular symmetry.

This annularly closed portion comprises an intermediate annularly closed region and two outer annularly closed regions arranged, in the radial direction, on both sides of the intermediate annularly closed region. Thus, each of these three regions surrounds the central domed portion. Preferably, the intermediate annularly closed region is directly connected between the two outer annularly closed regions, preferably by means of appropriately curved transition regions of the outer annularly closed regions, such that when traveling in the radial direction from the outer edge of the central domed portion over the annularly closed portion to the sidewall, any changes of the radius of curvature are continuous and not step-like.

In any case, in this embodiment one of the two outer annularly closed regions is located directly adjacent, or is connected to, the sidewall and the other of the two outer annularly closed regions is located directly adjacent, or connected to, the central domed portion.

Further, in cross section in the radial direction the bottom wall has an inwardly curved C-shape in the intermediate annularly closed region and an outwardly curved C-shape in each of the two outer annularly closed regions. In other words, in the intermediate annularly closed region on the one hand and in the two outer annularly closed regions on the other hand the centers of curvature are on opposite sides of the annularly closed portion of the bottom wall. Preferably, the radius of curvature is greater in the intermediate annularly closed region, for example about 1.0 to 2.0 mm, in particular 1.5 mm, than in each of the two outer annularly closed regions, for example about 0.1 to 1.0 mm, in particular 0.5 mm.

In this manner, a radially outer border region of the central domed portion and/or at least a part of the outer annularly closed region located directly adjacent the central domed portion define an annularly closed ridge which constitutes a stand portion of the canister, i.e. the lowest point of the canister when it is arranged with the bottom wall pointing downwardly.

Further, and more importantly, an annularly closed inward depression region is provided in a border region of the bottom wall, which serves to strengthen the bottom wall and the transition between the bottom wall and the sidewall, thereby allowing for higher internal pressures. Also, it has been found that this depression region allows for a further considerable particular decrease of the wall thickness values of the bottom wall and the sidewall.

Specifically, the thickness of the sidewall may preferably be in the range of 0.15 to 0.3 mm, more preferably in the range of 0.16 to 0.25 mm, even more preferably in the range of 0.17 to 0.25 mm, still more preferably in the range of 0.18 to 0.25 mm, still even more preferably in the range of 0.19 to 0.25 mm, and most preferably in the range of 0.2 to 0.25 mm. Also, the thickness of the bottom wall may be in any of the ranges mentioned above, preferably in the range of 0.36 to 0.4 mm.

In a preferred embodiment the top cover comprises a metering valve assembly sealingly carried by the top cover for metering a dose of a medicament contained in the interior cavity of the canister. The metering valve may have the construction outlined above in connection with the description of the prior art canisters.

In a preferred embodiment the canister is part of an MDI of the construction described in detail above. In other words, this embodiment relates to an MDI including a canister in accordance with any of the embodiments described in this application.

In any case, the canister has a diameter of 15 to 30 mm and a length of 20 to 75 mm, measured from the lowest point of the bottom wall along the longitudinal axis to the top cover. Further, the interior volume of the canister may be 7 to 30 ml brim full.

A canister having the above construction can be produced advantageously by means of a method which includes providing a circular metal blank, deep drawing a cup-shaped container using one or more deep drawing steps, possibly with intermediate annealing steps, and ironing the sidewall in order to reduce the wall thickness thereof. The thickness of the circular metal blank, the dies and the process parameters are selected such that the shape and the thickness values defined above are obtained for the cup-shaped component. Subsequently, the top cover may be secured to the cup-shaped component, e.g. by means of crimping it around an opening or mouth of the sidewall opposite the bottom wall. Preferably, a seal or gasket is provided between a portion of the top cover and a portion of the sidewall.

In the following an embodiment of the invention is explained in detail with reference to the drawings.

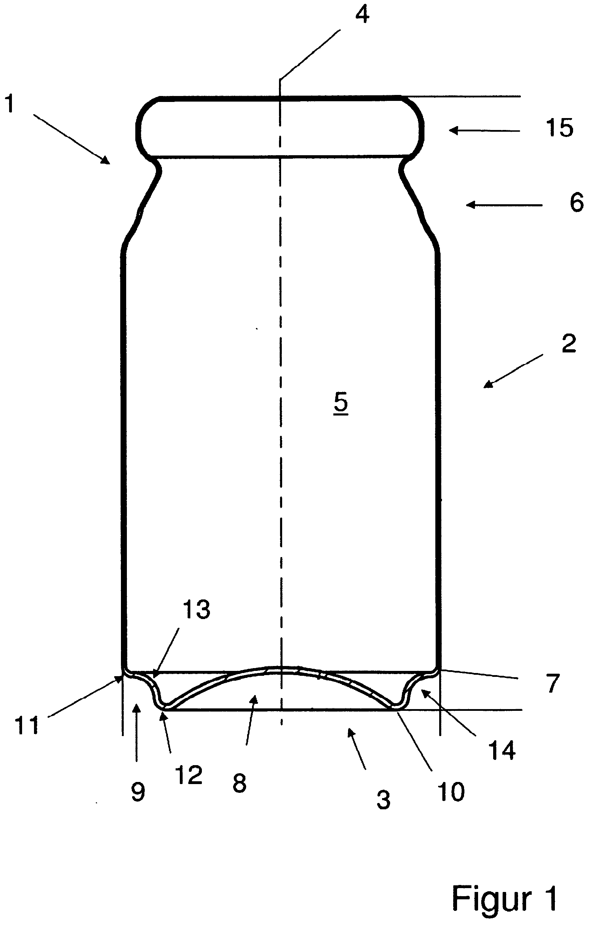

Figure 1 shows a cross sectional view of a canister according to the present invention.

The canister 1 generally comprises a sidewall 2, a bottom wall 3, from which the sidewall 2 extends upwardly, and a top cover 15 secured to and closing the opposite end of the sidewall 2. The sidewall 2 is cylindrical with a circular cross section and defines a longitudinal axis 4. Together, the sidewall 2, the bottom wall 3 and the top cover 15 define an interior cavity 5 of the canister 1.

Near its upper end the sidewall 2 comprises a reduced diameter section 6 forming a necked portion to which the top cover 15 is secured by crimping. As noted above, the details of the top cover 15 are not essential to the embodiment shown.

The bottom wall 3 and the sidewall 2 are formed integrally in one piece from sheet metal, and the bottom wall 3 is directly connected to the lower end 7 of the sidewall 2.

In the radial direction the bottom wall 3 comprises two distinct portions, namely a central inwardly domed portion 8 and an annularly closed portion 9 completely surrounding and directly connected to the domed portion 8. The annularly closed portion 9, in turn, comprises three distinct regions in the radial direction which are distinguished by the radius of curvature. Directly adjacent the lower end 7 of the sidewall and the outer border 10 of the central domed portion 8 there are provided two outer annularly closed regions 11 and 12, respectively, and between these two outer annularly closed regions 11 and 12 an intermediate annularly closed region 13 is provided which is directly connected to the outer annularly closed regions 11 and 12. Each of the regions 11, 12 and 13 is C-shaped in the cross sectional view illustrated, and they are arranged such that the change of the radius of curvature is continuous when moving from the sidewall 2 in the radial direction over the annularly closed portion 9 to the central domed portion 8.

As can be seen in the Figure, the centers of curvature of the two outer annularly closed regions 11 and 12 are located inside the interior cavity 5 of the canister 1, and the center of curvature of the intermediate annularly closed region 13 is located outside the canister 1. The radius of curvature of the intermediate annularly closed region 13 is preferably about 1 mm and larger than the radius of curvature of the two outer annularly closed regions 11 and 12, which is preferably 0.5 mm.

The intermediate annularly closed region 13 defines an annularly closed depression 14 in the edge region of the bottom wall 3 which serves to strengthen the bottom wall 3 and the transition between the bottom wall 3 and the sidewall 2.

The outer border 10 of the central domed portion 8 together with the adjacent outer annularly closed region 12 forms a stand portion of the canister 1.

Throughout the entire sidewall 2 the wall thickness is smaller than the minimum wall thickness of the bottom wall 3, in particular smaller than the minimum wall thickness of the central domed portion 8. For example, the maximum wall thickness of the sidewall 2 is 0.2 mm and the wall thickness of the bottom wall 3 is 0.38 mm. It has been found by experiments that this canister 1 is able to safely withstand interior pressures of 20 bar while achieving material savings of 46% as compared to a standard 14 ml (brim full) canister having an essentially constant wall thickness of about 0.47 mm.

## Claims

1. A canister for a metered dose inhaler for storing a propellant and a medicament under pressure and dispensing said medicament, said canister (1) comprising an interior cavity (5) defined by
- a tubular sidewall (2) defining a longitudinal axis (4) of the canister (1), and
- a bottom wall (3) and a top cover (15) closing opposite longitudinal ends of the sidewall (2) such that the sidewall (2) extends along the longitudinal axis (4) between the bottom wall (3) and the top cover (15),
- wherein the bottom wall (3) comprises a central inwardly domed portion (8),
wherein the wall thickness of the sidewall (2) is smaller than the wall thickness of the bottom wall (3), and wherein the thickness of the bottom wall (3) is in the range of 0.25 to 0.47 mm and the thickness of the sidewall (2) is in the range of 0.15 to 0.42 mm,
**characterized in that** the canister (1) has a diameter of 15 to 30 mm and a length of 20 to 75 mm, measured from the lowest point of the bottom wall (3) along the longitudinal axis (4) to the top cover (15).

2. The canister according to claim 1, wherein the sidewall (2) is cylindrical.

3. The canister according to claim 1 or claim 2, wherein the sidewall (2) and the bottom wall (3) are integrally formed in one piece.

4. The canister according to any of the preceding claims, wherein the thickness of the sidewall (2) is in the range of 0.18 to 0.36 mm.

5. The canister according to claim 4, wherein the thickness of the sidewall (2) is in the range of 0.2 to 0.36 mm.

6. The canister according to claim 5, wherein the thickness of the sidewall (2) is in the range of 0.3 to 0.36 mm.

7. The canister according to claim 1 or claim 2, wherein the bottom wall (3) comprises an annularly closed portion (9) surrounding the central domed portion (8) and disposed between the central domed portion (8) and the sidewall (2),
- wherein the annularly closed portion (9) comprises an intermediate annularly closed region (13) and two outer annularly closed regions (11, 12) on both sides of the intermediate annularly closed region (13) in the radial direction,
- wherein one (11) of the two outer annularly closed regions (11, 12) is located directly adjacent the sidewall (2) and the other (12) of the two outer annularly closed regions (11, 12) is located directly adjacent the central domed portion (8), and
- wherein in cross section in the radial direction the bottom wall (3) has an inwardly curved C-shape (14) in the intermediate annularly closed region (13) and an outwardly curved C-shape in each of the two outer annularly closed regions (11, 12).

8. The canister according to claim 7, wherein the radius of curvature is greater in the intermediate annularly closed region (13) than in each of the two outer annularly closed regions (11, 12).

9. The canister according to claim 7 or claim 8, wherein the thickness of the sidewall (2) is in the range of 0.15 to 0.3 mm.

10. The canister according to claim 9, wherein the thickness of the sidewall (2) is in the range of 0.18 to 0.25 mm.

11. The canister according to claim 10, wherein the thickness of the sidewall (2) is in the range of 0.2 to 0.25 mm.

12. The canister according to any of claims 7 to 11, wherein the thickness of the bottom wall (3) is in the range of 0.36 to 0.4 mm.

13. The canister according to any of the preceding claims, wherein the top cover (15) comprises a metering valve assembly sealingly carried by the top cover (15) for metering a dose of said suspension or solution.

14. A method of producing a canister (1) according to any of the preceding claims comprising the following steps:
- providing a circular metal blank,
- deep drawing a cup-shaped container, and
- ironing the sidewall (2) in order to reduce the wall thickness thereof.

15. The method according to claim 14, further comprising the steps of providing a top cover (15) including a metering valve and attaching the top cover (15) to cup-shaped container.

## Patentansprüche

1. Behälter für einen Dosierinhalator (metered dose inhaler) zum Speichern eines Treibmittels und eines Medikaments unter Druck und zum Abgeben des Medikaments, wobei der Behälter (1) einen inneren Hohlraum umfasst, der definiert ist durch
- eine rohrförmige Seitenwand (2), die eine Längsachse (4) des Behälters (1) definiert, und
- eine Bodenwand (3) und eine obere Abdeckung (15), die gegenüberliegende longitudinale Enden der Seitenwand (2) schließen, sodass sich die Seitenwand (2) entlang der Längsache (4) zwischen der Bodenwand (3) und der oberen Abdeckung (15) erstreckt,
- wobei die Bodenwand (3) einen zentralen nach innen gewölbten Teil (8) umfasst,
wobei die Wanddicke der Seitenwand (2) geringer ist als die Wanddicke der Bodenwand (3) und wobei die Dicke der Bodenwand (3) im Bereich von 0,25 bis 0,47 mm und die Dicke der Seitenwand (2) im Bereich von 0,15 bis 0,42 mm liegt,
**dadurch gekennzeichnet, dass** der Behälter (1) einen Durchmesser von 15 bis 30 mm und eine Länge von 20 bis 75 mm aufweist, gemessen von dem untersten Punkt der Bodenwand (3) entlang der Längsachse (4) zur oberen Abdeckung (15).

2. Behälter nach Anspruch 1, wobei die Seitenwand (2) zylindrisch ist.

3. Behälter nach Anspruch 1 oder Anspruch 2, wobei die Seitenwand (2) und die Bodenwand (3) einstückig aus einem Stück gebildet sind.

4. Behälter nach einem der vorhergehenden Ansprüche, wobei die Dicke der Seitenwand (2) im Bereich von 0,18 bis 0,36 mm liegt.

5. Behälter nach Anspruch 4, wobei die Dicke der Seitenwand (2) im Bereich von 0,2 bis 0,36 mm liegt.

6. Behälter nach Anspruch 5, wobei die Dicke der Seitenwand (2) im Bereich von 0,3 bis 0,36 mm liegt.

7. Behälter nach Anspruch 1 oder Anspruch 2, wobei die Bodenwand (3) einen ringförmig geschlossenen Teil (9) umfasst, der den zentralen gewölbten Teil (8) umgibt und zwischen dem zentralen gewölbten Teil (8) und der Seitenwand (2) vorliegt,
- wobei der ringförmig geschlossene Teil (9) einen ringförmig geschlossenen Zwischenbereich (13) und zwei äußere ringförmig geschlossene Bereiche (11, 12) an beiden Seiten des ringförmig geschlossenen Zwischenbereichs (13) in Radialrichtung umfasst,
- wobei einer (11) der beiden äußeren ringförmig geschlossenen Bereiche (11, 12) unmittelbar benachbart zu der Seitenwand (2) vorliegt und der andere (12) der beiden äußeren ringförmig geschlossenen Bereiche (11, 12) unmittelbar benachbart zu dem zentralen gewölbten Bereich (8) vorliegt und
- wobei die Bodenwand (3) in Radialrichtung im Querschnitt eine nach innen gewölbte C-Form (14) in dem ringförmig geschlossenen Zwischenbereich (13) aufweist und eine nach außen gewölbte C-Form in jedem der beiden äußeren ringförmig geschlossenen Bereiche (11, 12) aufweist.

8. Behälter nach Anspruch 7, wobei der Radius der Wölbung in dem ringförmig geschlossenen Zwischenbereich (13) größer ist als in jedem der zwei äußeren ringförmig geschlossenen Bereiche (11, 12).

9. Behälter nach Anspruch 7 oder Anspruch 8, wobei die Dicke der Seitenwand (2) im Bereich von 0,15 bis 0,3 mm liegt.

10. Behälter nach Anspruch 9, wobei die Dicke der Seitenwand (2) im Bereich von 0,18 bis 0,25 mm liegt.

11. Behälter nach Anspruch 10, wobei die Dicke der Seitenwand (2) im Bereich von 0,2 bis 0,25 mm liegt.

12. Behälter nach einem der Ansprüche 7 bis 11, wobei die Dicke der Bodenwand (3) im Beriech von 0,36 bis 0,4 mm liegt.

13. Behälter nach einem der vorhergehenden Ansprüche, wobei die obere Abdeckung (15) eine Dosierventilanordnung aufweist, die dichtend von der oberen Abdeckung (15) getragen wird, um eine Dosis der Suspension oder Lösung abzugeben.

14. Verfahren zur Herstellung eines Behälters (1) gemäß einem der vorhergehenden Ansprüche, bei dem
- ein runder Metallrohling zur Verfügung gestellt wird,
- ein becherförmiger Behälter tiefgezogen wird und
- die Seitenwand (2) geplättet wird, um deren Wanddicke zu verringern.

15. Verfahren nach Anspruch 14, bei dem ferner eine obere Abdeckung (15) zur Verfügung gestellt wird, die ein Dosierventil aufweist, und die obere Abdeckung (15) an den becherförmigen Behälter angebracht wird.

## Revendications

1. Cartouche pour aérosol-doseur destinée à contenir un gaz propulseur et un médicament sous pression et à distribuer ledit médicament, ladite cartouche (1) comportant une cavité intérieure (5) définie par :
- une paroi latérale tubulaire (2) définissant un axe longitudinal (4) de la cartouche (1), et
- une paroi de fond (3) et un couvercle au sommet (15) fermant des extrémités longitudinales espacées de la paroi latérale (2) d'une manière telle que la paroi latérale (2) s'étend suivant l'axe longitudinal (4) entre la paroi de fond (3) et le couvercle (15),
- la paroi de fond (3) comprenant une partie centrale (8) bombée vers l'intérieur,
l'épaisseur de la paroi latérale (2) étant plus petite que l'épaisseur de la paroi de fond (3), et l'épaisseur de la paroi de fond (3) étant de 0,25 à 0,37 mm et l'épaisseur de la paroi latérale (2) étant de 9,15 à 0,42 mm,
la cartouche (1) étant **caractérisée en ce qu'**elle a un diamètre de 15 à 30 mm et une longueur de 20 à 75 mm, mesurée depuis le point le plus bas de la paroi de fond (3) sur l'axe longitudinal. (4) jusqu'au couvercle (15).

2. Cartouche selon la revendication 1, dans laquelle la paroi latérale (2) est cylindrique.

3. Cartouche selon la revendication 1 ou 2, dans laquelle la paroi latérale (2) et la paroi de fond (3) sont formées d'un seul tenant.

4. Cartouche selon l'une quelconque des revendications précédentes, dans laquelle l'épaisseur de la paroi latérale (2) est de 0,18 à 0,36 mm.

5. Cartouche selon l'une quelconque des revendications précédentes, dans laquelle l'épaisseur de la paroi latérale (2) est de 0,2 à 0,36 mm.

6. Cartouche selon l'une quelconque des revendications précédentes, dans laquelle l'épaisseur de la paroi latérale (2) est de 0,3 à 0,36 mm.

7. Cartouche selon la revendication 1 ou la revendication 2, dans laquelle la paroi de fond (3) comprend une partie à fermeture annulaire (9) entourant la partie centrale bombée (8) et disposée entre la partie centrale bombée (8) et la paroi latérale (2),
- la partie à fermeture annulaire (9) comprenant une région intermédiaire à fermeture annulaire (13) et deux régions extérieures à fermeture annulaire (11, 12) de part et d'autre de la région intermédiaire à fermeture annulaire (13) dans la direction radiale,
- une première (11) des deux régions extérieures à fermeture annulaire (11, 12) étant directement adjacente à la paroi latérale (2) et l'autre (12) des deux régions extérieures à fermeture annulaire (11, 12) étant directement adjacente à la partie centrale bombée (8), et
- en coupe transversale dans la direction radiale, la paroi de fond (3) ayant une forme de C incurvé vers l'intérieur (14) dans la région intermédiaire à fermeture annulaire (13) et une forme de C incurvé vers l'extérieur dans chacune des deux régions extérieures à fermeture annulaire (11, 12).

8. Cartouche selon la revendication 7, dans laquelle le rayon de courbure est plus grand dans la région intermédiaire à fermeture annulaire (13) que dans chacune des deux régions extérieures à fermeture annulaire (11, 12).

9. Cartouche selon la revendication 7 ou la revendication 8, dans laquelle l'épaisseur de la paroi latérale (2) est de 0,15 à 0,3 mm.

10. Cartouche selon la revendication 9, dans laquelle l'épaisseur de la paroi latérale (2) est de 0,18 à 0,25 mm

11. Cartouche selon la revendication 10, dans laquelle l'épaisseur de la paroi latérale (2) est de 0,2 à 0,25 mm.

12. Cartouche selon l'une quelconque des revendications 7 à 11, dans laquelle l'épaisseur de la paroi de fond (3) est de 0,36 à 0,4 mm.

13. Cartouche selon l'une quelconque des revendications précédentes, dans laquelle le couvercle (15) au sommet comprend un système de valve doseuse portée d'une manière étanche par le couvercle (15) pour mesurer une dose de ladite suspension ou solution.

14. Procédé de fabrication de cartouche (1) selon l'une quelconque des revendications précédentes, comportant les étapes suivantes :
- réalisation d'une ébauche circulaire en métal,
- emboutissage d'un récipient en forme de gobelet, et
- étirage de la paroi latérale (2) afin de réduire l'épaisseur de celle-ci.

15. Procédé selon la revendication 14, comportant en outre les étapes de réalisation d'un couvercle (15) comprenant une valve doseuse et la fixation du couvercle (15) au sommet du récipient en forme de gobelet.
